# EUROPÄISCHE PATENTSCHRIFT

(11) **EP 2 528 508 B1**
(45) Veröffentlichungstag und Bekanntmachung des Hinweises auf die Patenterteilung: **04.05.2016**
(21) Anmeldenummer: 11713205.0
(22) Anmeldetag: 22.03.2011
(51) Int. Cl.: A61B 6/00, A61B 6/03, F16C 32/04, F16C 39/06, H02K 7/09

(54) **VORRICHTUNG ZUR LAGERUNG UND ZUM ANTRIEB EINES ROTIERBAREN TEILS EINER GANTRY EINES COMPUTERTOMOGRAPHIEGERÄTES UND COMPUTERTOMOGRAPHIEGERÄT**
DEVICE FOR MOUNTING AND DRIVING A ROTATABLE PART OF A GANTRY OF A COMPUTER TOMOGRAPHY UNIT AND COMPUTER TOMOGRAPHY UNIT
DISPOSITIF POUR SOUTENIR ET POUR ENTRAÎNER UNE PARTIE ROTATIVE D'UN PORTIQUE MOBILE D'UN TOMODENSITOMÈTRE ET TOMODENSITOMÈTRE

(30) Priorität: 15.04.2010 DE 102010015061
(43) Veröffentlichungstag der Anmeldung: 05.12.2012
(73) Patentinhaber: Siemens Aktiengesellschaft, 80333 München (DE)
(72) Erfinder: KALENYAK, Johann, 96110 Schesslitz (DE); MÜLLER, Hans-Jürgen, 91362 Pretzfeld (DE)
(86) Internationale Anmeldenummer: PCT/EP2011/054334
(87) Internationale Veröffentlichungsnummer: WO 2011/128187

(56) Entgegenhaltungen:
- EP-A1- 0 549 912
- EP-A1- 0 657 915
- EP-A1- 0 676 911
- WO-A1-2005/102171
- WO-A2-2010/026523
- US-A- 4 723 259
- US-A- 5 548 629
- US-A1- 2005 116 558

## Beschreibung

Vorrichtung zur Lagerung und zum Antrieb eines rotierbaren Teils einer Gantry eines Computertomographiegerätes und Computertomographiegerät

Die Erfindung betrifft eine Vorrichtung zur Lagerung und zum Antrieb eines rotierbaren Teils einer eine Systemachse aufweisenden Gantry eines Computertomographiegerätes relativ zu einem stationären Teil der Gantry des Computertomographiegerätes. Die Erfindung betrifft außerdem ein Computertomographiegerät, das eine solche Vorrichtung umfasst.

Computertomographiegeräte der dritten Generation weisen eine Gantry mit einem stationären Teil und einem relativ zu dem stationären Teil rotierbaren Teil auf. Der rotierbare Teil hat die Form einer Trommel an der Komponenten des Computertomographiegerätes wie die Röntgenstrahlenquelle, der Röntgenstrahlendetektor, ein Steuerungssystem etc. angeordnet sind, welche sich im Betrieb des Computertomographiegerätes um einen in einem Messfeld angeordneten Patienten drehen. Eine komplett bestückte Trommel erreicht bei einem System mit nur einer Röntgenstrahlenquelle und einem Röntgenstrahlendetektor eine Masse von ca. 800 kg bis 900 kg und rotiert im Betrieb mit einer Umdrehungsgeschwindigkeit von bis zu 240 U/min.

Zur Lagerung der Trommel relativ zu dem stationären Teil der Gantry werden heutzutage Wälzlager in unterschiedlichen Ausführungen eingesetzt. Die Wälzlager unterliegen jedoch einem gewissen Verschleiß und müssen regelmäßig gewartet, z.B. nachgeschmiert werden. Darüber hinaus steigt der von den Wälzlagern ausgehende Geräuschpegel mit zunehmender Drehzahl, dass er als störend empfunden werden kann und zusätzliche Maßnahmen zu dessen Reduzierung notwendig werden können. Der Antrieb der Trommel erfolgt in der Regel über einen Treibriemen.

Aus dem Stand der Technik sind verschiedene Bildgebungseinrichtungen bekannt, welche einen stationären Teil, einen rotierbaren Teil und eine Einheit zur Lagerung und zur Rotation des rotierbaren Teils 2 relativ zu dem stationären Teils aufweisen. US 4,723,259 A offenbart beispielsweise ein mechanisches Lager, welcher einen Antrieb mit einem elektromagnetischen Motor vorsieht. WO 2005/102171 A1 offenbart weiterhin ein Computertomographiegerät wobei eine Art Luft- oder Fluidkissen-Lager zur Lagerung vorgesehen ist. Schließlich offenbaren beispielsweise WO 2010/026523 A2, EP 0 676 911 A1 und US 5,548,629 A eine magnetische Lagerung des rotierbaren Teils und einen elektromagnetischen Antrieb.

Der Erfindung liegt die Aufgabe zugrunde, eine Vorrichtung und ein Computertomographiegerät der eingangs genannten Art derart anzugeben, dass die Lagerung und der Antrieb eines rotierbaren Teils einer Gantry relativ zu eine stationären Teil einer Gantry eines Computertomographiegerätes verbessert ist.

Nach der Erfindung wird diese Aufgabe gelöst durch eine Vorrichtung gemäß dem Anspruch 1.

Durch die magnetische Lagerung des rotierbaren Teils der Gantry relativ zu dem stationären Teil der Gantry treten keine mechanischen Berührungen mehr zwischen Lagerteilen auf. Die magnetische Lagerung ist reibungs- und verschleißfrei, so dass ein Nachschmieren oder ein Nachfetten wie bei Wälzlagern nicht nötig ist. Auch die Geräuschentwicklung im Betrieb ist bei einer magnetischen Lagerung deutlich geringer als bei Verwendung eines Wälzlagers.

Vorzugsweise sind die Mittel zum elektromagnetischen Antrieb des rotierbaren Teils der Gantry relativ zu dem stationären Teil der Gantry und die Mittel zur magnetischen Lagerung des rotierbaren Teils der Gantry relativ zu dem stationären Teil der Gantry in einer Einheit, insbesondere einer Baueinheit, kombiniert.

Nach einer weiteren Variante der Erfindung umfassen die Mittel zur magnetischen Lagerung des rotierbaren Teils der Gantry relativ zu dem stationären Teil der Gantry wenigstens einen Permanentmagneten, wenigstens einen eine Spule aufweisenden Elektromagneten und/oder wenigstens ein Element aus einem ferromagnetischen Material und die Mittel zum elektromagnetischen Antrieb des rotierbaren Teils der Gantry relativ zu dem stationären Teil der Gantry wenigstens einen eine Spule aufweisenden Elektromagneten. Das Radial- und das Axiallager können als rein passive Magnetlager unter entsprechender Anordnung von Permanentmagneten und Elementen aus einem ferromagnetischen Material auf dem rotierbaren und dem stationären Teil der Gantry relativ zueinander realisiert werden, wobei entweder die zwischen Permanentmagneten auftretenden abstoßenden oder anziehenden Kräfte oder die Anziehungskräfte zwischen Permanentmagneten und ferromagnetischen Materialien ausgenutzt werden. Bevorzugt weisen das Radial- und das Axiallager aber auch Spulen umfassende Elektromagnete auf, um zur Stabilisierung der Magnetlager durch Variation der durch die Spulen der Elektromagnete fließenden Ströme das Magnetfeld und damit die aktuell wirkenden Kräfte in dem jeweiligen Magnetlager verändern zu können. Hierzu ist eine entsprechende Regelung erforderlich, die dafür sorgt, dass die jeweils benötigten Lagerkräfte zur Verfügung stehen.

Die in der Regel mehreren, jeweils wenigstens eine Spulen aufweisenden Elektromagnete der Mittel zum elektromagnetischen Antrieb des rotierbaren Teils der Gantry relativ zu dem stationären Teil der Gantry sind zur Erzeugung eines elektromagnetischen Drehfeldes zum Antrieb des rotierbaren Teils relativ zu dem stationären Teil der Gantry erforderlich und müssen hierzu entsprechend angesteuert werden. Die Mittel zum elektromagnetischen Antrieb können im Übrigen auch Permanentmagnete und/oder Elementen aus einem ferromagnetischen Material aufweisen.

Nach einer Ausführungsform der Erfindung weisen das Radiallager und/oder die Mittel zum elektromagnetischen Antrieb eine erste, radial außenliegende ringförmige Radialanordnung von Permanentmagneten, Elektromagneten und/oder Elementen aus einem ferromagnetischen Material, welche dem stationären Teil der Gantry zugeordnet ist, und eine zweite radial innenliegende ringförmige Radialanordnung von Permanentmagneten, Elektromagneten und/oder Elementen aus einem ferromagnetischen Material auf, welche dem rotierbaren Teil der Gantry zugeordnet ist, wobei sich zwischen der ersten und der zweiten ringförmigen Radialanordnung ein ringförmiger Radiallagerspalt befindet.

Nach einer weiteren Ausführungsform der Erfindung weist das Axiallager eine erste ringförmige Axialanordnung von Permanentmagneten, Elektromagneten und/oder Elementen aus einem ferromagnetischen Material auf, welche dem stationären Teil der Gantry zugeordnet ist, und eine zweite ringförmige Axialanordnung von Permanentmagneten, Elektromagneten und/oder Elementen aus einem ferromagnetischen Material auf, welche dem rotierbaren Teil der Gantry zugeordnet ist, welche erste und zweite Axialanordnung unter Einschluss eines ringförmigen Axiallagerspalts axial in Richtung der Systemachse relativ zueinander versetzt sind.

Eine Variante der Erfindung sieht vor, dass die Vorrichtung bzw. die Baueinheit aus Mitteln zur magnetischen Lagerung und Mitteln zum elektromagnetischen Antrieb des rotierbaren Teils relativ zu dem stationären Teil der Gantry Messmittel zur Ermittlung der Änderung der Weite des ringförmigen Radiallagerspalts und/oder des ringförmigen Axiallagerspalts aufweist. Um eine funktionelle und störungsfreie magnetische Lagerung realisieren zu können, muss die Weite der Lagerspalte im Wesentlichen konstant sein bzw. gehalten werden. Die Weite eines Lagerspaltes ist quasi die Regelgröße bei der Regelung der Lagerkräfte. Die Weite wird dabei vorzugsweise berührungslos mit den Messmitteln bestimmt. Die Weite wird in der Regel mindestens an zwei, vorzugsweise um ca. 90° relativ zueinander versetzten Stellen der ringförmigen Lagerspalte bestimmt. Die Weite eines Lagerspaltes muss im Übrigen konstruktionsbedingt nicht über den ganzen Lagerspalt immer gleich sein. Vielmehr kann der Lagerspalt eine Art Profil aufweisen, so dass sich an verschiedenen Stellen des Lagerspaltes verschiedene Weiten des Lagerspaltes ergeben. In einem solchen Fall wird die Weite des Lagerspaltes jeweils an einer bestimmten Stelle des Lagerspaltes ermittelt und zur Regelung verwendet.

Vorzugsweise weisen die Messmittel wenigstens einen Hall-Sensor und/oder einen induktiv oder kapazitiv arbeitenden Sensor auf.

Gemäß einer Variante der Erfindung erfolgt im Falle der Verwendung von Elektromagneten für die magnetische Lagerung die Ermittlung der Änderung der Weite des Radiallagerspalts und/oder des Axiallagerspalts basierend auf der Beeinflussung der Induktivität der Spule eines oder mehrerer Elektromagnete. So beeinflusst beispielsweise eine Positionsänderung der innenliegenden Radialanordnung von Permanentmagneten, Elektromagneten und/oder Elementen aus einem ferromagnetischen Material des rotierbaren Teils der Gantry relativ zu der außenliegenden Radialanordnung die Induktivitäten der Elektromagneten der außenliegenden Radialanordnung. Dadurch ändern sich Strom- und Spannungswerte an den Elektromagneten der außenliegenden Radialanordnung, welche Änderungen zur Ermittlung der Weite des jeweiligen Lagerspalts ausgewertet werden.

Eine Ausgestaltung der Erfindung sieht vor, dass das Radiallager in einer ersten Ebene und die Mittel zum elektromagnetischen Antrieb des rotierbaren Teils der Gantry relativ zu dem stationären Teil der Gantry in einer zweiten, in Richtung der Systemachse relativ zu der ersten Ebene versetzten Ebene angeordnet sind.

Nach einer ersten solchen Variante der Integration weisen die erste, radial außenliegende ringförmige Radialanordnung Elektromagnete und die zweite radial innenliegende ringförmige Radialanordnung Permanentmagnete und/oder Elementen aus einem ferromagnetischen Material auf, wobei eine Gruppe von Elektromagneten der ersten Radialanordnung derart ansteuerbar ist und angesteuert wird, dass ein elektromagnetisches Drehfeld zum elektromagnetisches Antrieb des rotierbaren Teils der Gantry erzeugt wird.

Nach einer zweiten solchen Variante der Integration weisen die erste, radial außenliegende ringförmige Radialanordnung Permanentmagnete und/oder Elementen aus einem ferromagnetischen Material und die zweite radial innenliegende ringförmige Radialanordnung Elektromagnete auf, wobei eine Gruppe von Elektromagneten der zweiten Radialanordnung derart ansteuerbar ist und angesteuert wird, dass ein elektromagnetisches Drehfeld zum elektromagnetisches Antrieb des rotierbaren Teils der Gantry erzeugt wird.

Nach einer weiteren Variante der Erfindung weisen die erste, radial außenliegende ringförmige Radialanordnung und die zweite radial innenliegende ringförmige Radialanordnung Elektromagnete auf, wobei eine Gruppe von Elektromagneten der ersten und/oder der zweiten Radialanordnung derart ansteuerbar ist und angesteuert wird, dass ein elektromagnetisches Drehfeld zum elektromagnetisches Antrieb des rotierbaren Teils der Gantry erzeugt wird.

Gemäß einer Ausführungsform der Erfindung sind die zum elektromagnetischen Antrieb und/oder die zur magnetischen Lagerung vorgesehenen Elektromagnete der ersten und/oder der zweiten Radialanordnung jeweils in Segmenten gruppiert. Die Elektromagnete können jeweils in zwei oder mehr Segmenten gruppiert sein. Beispielsweise können die zum elektromagnetischen Antrieb vorgesehenen Elektromagnete in drei jeweils ca. 60° des Radiallagers abdeckenden Segmenten gruppiert sein, die jeweils durch ein ebenfalls einen 60°-Winkel abdeckendes Segment voneinander getrennt sind, in dem zur magnetischen Lagerung vorgesehene Elektromagnete gruppiert sind. Bei einer Vier-Segmentanordnung decken die zum elektromagnetischen Antrieb vorgesehenen Elektromagnete und die zur magnetischen Lagerung vorgesehenen Elektromagnete abwechselnd jeweils einen 45°-Winkel des Radiallagers ab, so dass vier Segmente für den Antrieb und vier Segmente für die Lagerung vorhanden sind. Weitere Segmentierungen sind ebenfalls möglich.

Eine weitere Ausführungsform der Erfindung sieht vor, dass die Vorrichtung bzw. die Baueinheit ein Wälzlager als Stützlager aufweist. Im Falle eines Stromausfalls übernimmt das Stützlager die Tragfunktion, so dass Beschädigungen der Vorrichtung vermieden werden.

Alternativ oder auch zusätzlich kann der Vorrichtung eine unterbrechungsfreie Stromversorgung (USV) zugeordnet sein.

Die der vorliegenden Erfindung zugrundeliegende Aufgabe wird auch gelöst durch ein Computertomographiegerät aufweisend eine der vorstehend beschriebenen Vorrichtungen.

Ausführungsbeispiele der Erfindung sind in den beigefügten schematischen Zeichnungen dargestellt. Es zeigen:
- FIG 1: ein Computertomographiegerät,
- FIG 2: den stationären und den rotierbaren Teil der Gantry des Computertomographiegerätes aus FIG 1 sowie eine Lager- und Antriebseinheit,
- FIG 3: eine Schnittdarstellung einer erste Ausführungsform einer Lager- und Antriebseinheit,
- FIG 4: eine Ansicht in Richtung der Pfeile IV des Schnitts aus FIG 3,
- FIG 5: eine Ansicht in Richtung der Pfeile IV des Schnitts aus FIG 3 für eine alternative Ausführungsform und
- FIG 6: eine Schnittdarstellung einer zweiten Ausführungsform einer Lager- und Antriebseinheit.

In den Figuren sind gleiche oder funktionsgleiche Elemente durchwegs mit gleichen Bezugszeichen versehen. Die Darstellungen in den Figuren sind schematisch und nicht zwingend maßstabsgetreu. Auf das Computertomographiegerät 1 wird im Folgenden und ohne Einschränkung der Allgemeinheit nur insoweit eingegangen als es zum Verständnis der Erfindung für erforderlich erachtet wird.

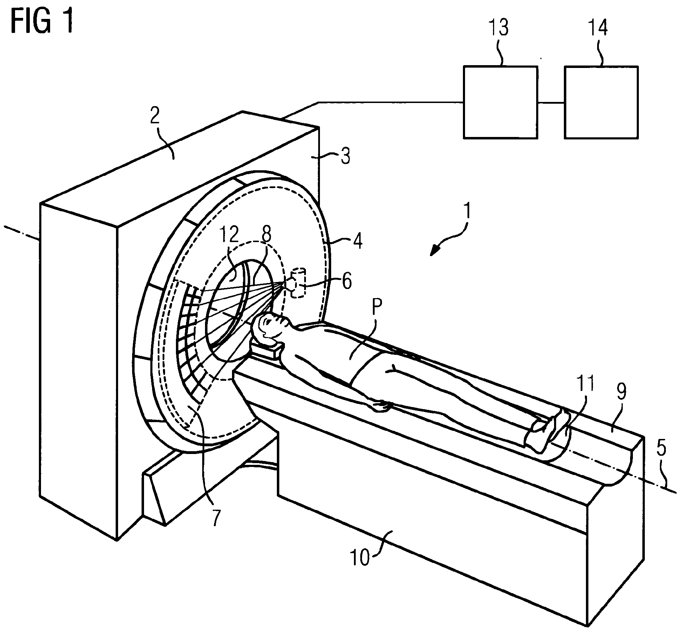

Das in FIG 1 gezeigte Computertomographiegerät 1 umfasst eine Gantry 2 mit einem stationären Teil 3 und mit einem um eine Systemachse 5 rotierbaren Teil 4. Der rotierbare Teil 4 weist im Falle des vorliegenden Ausführungsbeispiels der Erfindung ein Röntgensystem auf, welches eine Röntgenstrahlenquelle 6 und einen Röntgenstrahlendetektor 7 umfasst, die an dem rotierbaren Teil 4 einander gegenüberliegend angeordnet sind. Im Betrieb des Computertomographiegerätes 1 geht von der Röntgenstrahlenquelle 6 Röntgenstrahlung 8 in Richtung des Röntgenstrahlendetektors 7 aus, durchdringt ein Messobjekt und wird vom Röntgenstrahlendetektor 7 in Form von Messdaten bzw. Messsignalen erfasst.

Das Computertomographiegerät 1 weist des Weiteren eine Patientenliege 9 zur Lagerung eines zu untersuchenden Patienten P auf. Die Patientenliege 9 umfasst einen Liegensockel 10, an dem eine zur eigentlichen Lagerung des Patienten P vorgesehene Patientenlagerungsplatte 11 angeordnet ist. Die Patientenlagerungsplatte 11 ist derart relativ zu dem Liegensockel 10 in Richtung der Systemachse 5 verstellbar, dass sie zusammen mit dem Patienten P in die Öffnung 12 der Gantry 2 zur Aufnahme von 2D-Röntgenprojektionen von dem Patienten P, z. B. in einem Spiralscan, eingeführt werden kann. Die rechnerische Verarbeitung der mit dem Röntgensystem aufgenommenen 2D-Röntgenprojektionen bzw. die Rekonstruktion von Schichtbildern, 3D-Bildern oder eines 3D-Datensatzes basierend auf den Messdaten bzw. den Messsignalen der 2D-Röntgenprojektionen erfolgt mit einem Bildrechner 13 des Computertomographiegerätes 1, welche Schichtbilder oder 3D-Bilder auf einer Anzeigevorrichtung 14 darstellbar sind.

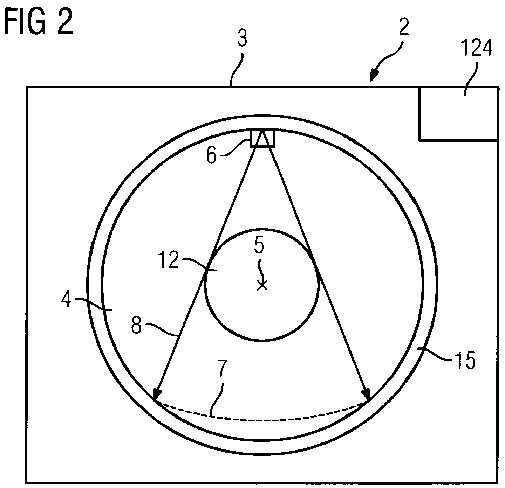

Damit der unter anderem mit dem Röntgensystem versehene rotierbare Teil 4 relativ zu dem stationären Teil 3 der Gantry rotieren kann ist eine drehbare Lagerung und ein Antrieb des rotierbaren Teils 4 erforderlich. Hierzu ist im Falle des vorliegenden Ausführungsbeispiels der Erfindung eine in FIG 2 schematisch veranschaulichte Lager- und Antriebseinheit 15 vorgesehen, welche Mittel zur magnetischen Lagerung und Mittel zum elektromagnetischen Antrieb des rotierbaren Teils 4 relativ zu dem stationären Teil 3 der Gantry 2 aufweist. Die Mittel zur magnetischen Lagerung und die Mittel zum elektromagnetischen Antrieb können grundsätzlich Permanentmagnete, Elektromagnete und/oder Elemente aus einem ferromagnetischen Material umfassen.

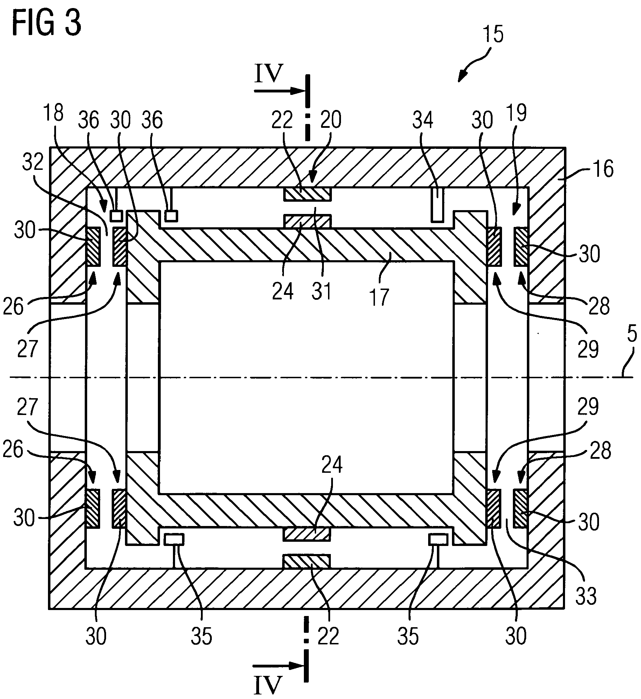

FIG 3 zeigt eine Schnittdarstellung einer ersten Ausführungsform einer derartigen Lager- und Antriebseinheit 15. Die Lager- und Antriebseinheit 15 umfasst im Falle des vorliegenden Ausführungsbeispiels der Erfindung ein erstes ringförmiges Trägerelement 16, welches am stationären Teil 3 angeordnet ist bzw. wird, und ein zweites ringförmiges Trägerelement 17, welches am rotierbaren Teil 4 angeordnet ist bzw. wird. An den ringförmigen Trägerelementen 16, 17 sind Mittel zur magnetischen Lagerung und Mittel zum elektromagnetischen Antrieb angeordnet, welche im Falle des vorliegenden Ausführungsbeispiels der Erfindung zwei magnetische Axiallager 18, 19 und ein magnetisches Radiallager 20 bilden, wobei in letzterem der elektromagnetische Antrieb integriert ist.

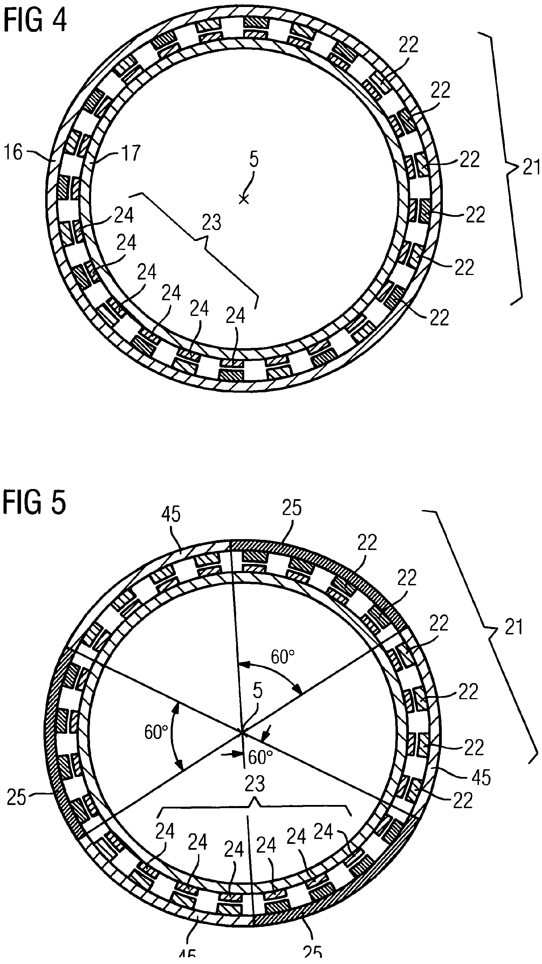

FIG 4 zeigt die Ansicht des Schnitts durch das Radiallager 20 in Richtung der Pfeile IV aus FIG 3. Das Radiallager 20 und die in das Radiallager 20 integrierten Mittel zum elektromagnetischen Antrieb weisen im Falle des in FIG 4 gezeigten Ausführungsbeispiels der Erfindung eine erste, radial außenliegende ringförmige Radialanordnung 21 von Elektromagneten 22 auf, welche jeweils wenigstens eine nicht explizit gezeigte Spule umfassen. Des Weiteren weisen das Radiallager 20 und die in das Radiallager 20 integrierten Mittel zum elektromagnetischen Antrieb eine zweite radial innenliegende ringförmige Radialanordnung 23 von Elementen 24 aus einem ferromagnetischen Material auf. Im Falle des in FIG 4 gezeigten Ausführungsbeispiels der Erfindung dienen 50% der Elektromagnete 22 der magnetischen Lagerung und 50% der Elektromagnete 22 dem elektromagnetischen Antrieb des rotierbaren Teils 4 relativ zu dem stationären Teil 3. Vorliegend ist jeder zweite Elektromagnet 22 der radial außenliegenden ringförmigen Radialanordnung 21 zur Erzeugung eines elektromagnetischen Drehfeldes vorgesehen, welche Elektromagnete 22 durch nicht explizit gezeigte Steuermittel derart angesteuert werden, dass in Zusammenwirken mit den Elementen 24 aus einem ferromagnetischen Material der rotierbare Teil 4 relativ zu dem stationären Teil 3 gedreht wird. Die übrigen Elektromagnete 22 dienen in Zusammenwirken mit den Elementen 24 aus einem ferromagnetischen Material der magnetischen Lagerung des rotierbaren Teils 4 relativ zu dem stationären Teil 3.

An Stelle der Elementen 24 aus einem ferromagnetischen Material kann insbesondere die zweite Radialanordnung auch Permanentmagnete oder beides aufweisen.

Nach einer alternativen Ausführungsform des mit Mitteln zum elektromagnetischen Antrieb versehenen Radiallagers 20 kann die radial außenliegende ringförmige Radialanordnung 21 Elementen aus einem ferromagnetischen Material und/oder Permanentmagnete und die radial innenliegende ringförmige Radialanordnung 23 Elektromagneten aufweisen, wobei, wie bereits zuvor beschrieben, jeder zweite Elektromagnet zur Erzeugung eines elektromagnetischen Drehfeldes vorgesehen ist, um bei entsprechender Ansteuerung mit Strom in Zusammenwirken mit den Elementen aus einem ferromagnetischen Material und/oder den Permanentmagneten den rotierbare Teil 4 relativ zu dem stationären Teil 3 in Drehung zu versetzen. Die übrigen Elektromagnete dienen in Zusammenwirken mit den Elementen aus einem ferromagnetischen Material und/oder den Permanentmagneten wiederum der magnetischen Lagerung des rotierbaren Teils 4 relativ zu dem stationären Teil 3. Bei dieser Variante müssen die für die Erzeugung des Drehfeldes erforderliche elektrische Energie sowie die Steuerungs- und Regelungssignale, sofern eine entsprechende Steuerungs- und Regelungseinheit nicht auf dem rotierbaren Teil 4 angeordnet ist, beispielsweise über Schleifringe auf den rotierbaren Teil 4 übertragen werden.

Eine weitere alternative Ausführungsform des mit Mitteln zum elektromagnetischen Antrieb versehenen Radiallagers 20 besteht darin, dass sowohl die radial außenliegende ringförmige Radialanordnung 21 als auch die radial innenliegende ringförmige Radialanordnung 23 Elektromagnete aufweist, wobei zumindest ein Teil der Elektromagnete der ersten und der zweiten Radialanordnung für die magnetische Lagerung vorgesehen sind und eine Gruppe von Elektromagneten der ersten und/oder der zweiten Radialanordnung derart ansteuerbar ist, dass ein elektromagnetisches Drehfeld zum elektromagnetisches Antrieb des rotierbaren Teils 4 der Gantry 2 erzeugt wird. Auch in diesem Fall müssen die für die Erzeugung des Drehfeldes erforderliche elektrische Energie sowie ggf. die Steuerungs- und Regelungssignale z.B. über Schleifringe auf den rotierbaren Teil 4 übertragen werden.

Die für den elektromagnetischen Antrieb vorgesehenen Elektromagneten 22 sowie die für die magnetische Lagerung vorgesehenen Elektromagnete 22 des Radiallagers 20 können auch jeweils in Segmenten gruppiert werden. FIG 5 zeigt eine derartige Segmentierung in Weiterentwicklung des in FIG 4 gezeigten Ausführungsbeispiels der Erfindung. Im Falle des in FIG 5 gezeigten Ausführungsbeispiels der Erfindung sind die zum Antrieb vorgesehenen Elektromagnete 22 in drei, jeweils einen 60°-Winkel abdeckenden Segmenten 25 angeordnet, wobei sich zwischen jedem Segment 25 ebenfalls ein 60°-Winkel befindet. Die in diesen Zwischensegmenten 45 angeordneten Elektromagnete 22 sind für die magnetische Lagerung vorgesehen. Andere Segmentanordnungen mit anderen Winkeln sind ebenfalls möglich. Eine derartige Segmentierung ist auch für die beschriebenen alternativen Ausführungsformen des mit Mitteln zum elektrischen Antrieb versehenen Radiallagers möglich.

Die Axiallager 18 und 19 sind im Prinzip wie das Radiallager 20 aufgebaut, weisen jedoch keine Mittel zum elektromagnetischen Antrieb auf.

Das Axiallager 18 weist eine erste ringförmige Axialanordnung 26 von Elektromagneten 30 und ggf. von Permanentmagneten und/oder von Elementen aus einem ferromagnetischen Material, welche an dem ersten ringförmigen Trägerelement 16 befestigt sind und dem stationären Teil 3 der Gantry 2 zugeordnet sind, und eine zweite ringförmige Axialanordnung 27 von Elektromagneten 30 und ggf. von Permanentmagneten und/oder von Elementen aus einem ferromagnetischen Material, welche an dem zweiten ringförmigen Trägerelement 17 befestigt sind und dem rotierbaren Teil 4 der Gantry 2 zugeordnet sind, auf.

Der Aufbau des Axiallagers 19 entspricht dem Aufbau des Axiallagers 18. Das Axiallager 19 weist also auch eine erste ringförmige Axialanordnung 28 von Elektromagneten 30 und ggf. von Permanentmagneten und/oder von Elementen aus einem ferromagnetischen Material, welche an dem ersten ringförmigen Trägerelement 16 befestigt sind und dem stationären Teil 3 der Gantry 2 zugeordnet sind, und eine zweite ringförmige Axialanordnung 29 von Elektromagneten 30 und ggf. von Permanentmagneten und/oder von Elementen aus einem ferromagnetischen Material, welche an dem zweiten ringförmigen Trägerelement 17 befestigt sind und dem rotierbaren Teil 4 der Gantry 2 zugeordnet sind, auf.

Um einen störungsfreien Betrieb der Magnetlager gewährleisten zu können, müssen stets die Weite des Radiallagerspaltes 31 und die Weiten der Axiallagerspalte 32 und 33 ermittelt werden. Hierzu sind im Falle des vorliegenden Ausführungsbeispiels der Erfindung Messmittel in Form von Hallsensoren vorgesehen. Die Weite eines Lagerspaltes muss dabei nicht direkt ermittelt werden, vielmehr kann diese aus der radialen bzw. axialen Position des ringförmigen Trägerelements 17 bzw. des rotierbaren Teils 4 berechnet werden. Weicht die Weite eines Lagerspaltes von ihrer Sollweite ab, muss die Weite wieder auf die Sollweite durch eine entsprechende Regelung der Spulenströme der hierfür relevanten Elektromagnete einstellt werden. Im Falle des vorliegenden Ausführungsbeispiels der Erfindung sind zur Bestimmung der Weite des Radiallagerspaltes zwei um ca. 90° radial versetzt zueinander angeordnete Hallsensoren 34 vorgesehen, von denen in FIG 3 nur einer dargestellt ist. Aus den Messwerten der Hallsensoren 34 kann aufgrund des definierten und bekannten Aufbaus der Lager- und Antriebeseinheit 15 die Weite des Radiallagerspaltes 31 durch eine Steuerungs- und Regelungseinheit ermittelt und zur Steuerung- und Regelung der Spulenströme der für die radiale Lagerung relevanten Elektromagnete verwendet werden.

In entsprechender Weise werden die Weiten der Axiallagerspalte 32 und 33 ermittelt. In FIG 3 sind eine erste Anordnung von Hallsensoren 35 und eine zweite Anordnung von Hallsensoren 36 gezeigt. Bevorzugt sind wenigstens zwei erste Anordnungen von Hallsensoren 35 und/oder wenigstens zwei zweite Anordnungen von Hallsensoren 36 um ca. 90° relativ zueinander radial versetzt zur Aufnahme von Messwerten vorhanden. Aus den Messwerten der Hallsensoren 35 und/oder der Hallsensoren 36 können die Weite des Axiallagerspaltes 32 sowie die Weite des Axiallagerspaltes 33 durch eine Steuerungs- und Regelungseinheit ermittelt und zur Steuerung- und Regelung der Spulenströme der für die axiale Lagerung relevanten Elektromagnete verwendet werden.

Alternativ erfolgt die Ermittlung der Weiten der Lagerspalte 31 bis 33 ohne zusätzliche Sensorik nur durch die Auswertung der Änderungen von Strom- und Spannungswerten von Elektromagneten. In Folge einer Lageänderung des ringförmigen Trägerelementes 17 bzw. des rotierbaren Teils 4 relativ zu dem ringförmigen Trägerelement 16 bzw. dem stationären Teil 3 werden die Induktivitäten der für die magnetische Lagerung relevanten Elektromagnete beeinflusst, wodurch sich die Strom- und Spannungswerte an den relevanten Elektromagneten ändert. Durch die Auswertung dieser Strom- und Spannungswerte können jeweils die Weiten der Lagerspalte ermittelt und für die Steuer- und Regelung der Spulenströme der relevanten Elektromagnete verwendet werden.

Hallsensoren können im Übrigen auch für die Rotationskontrolle verwendet werden, wobei ein Hallsensor verwendet werden kann, um die Rotationsposition des ringförmigen Trägerelementes 17 relativ zu dem ringförmigen Trägerelementes 16 zu erfassen. Ein oder zwei weitere Hallsensoren können vorgesehen sein, um die Rotationsrichtung zu erfassen. Auch diese Sensoren sind bevorzugt in die Lager -und Antriebseinheit 15 integriert.

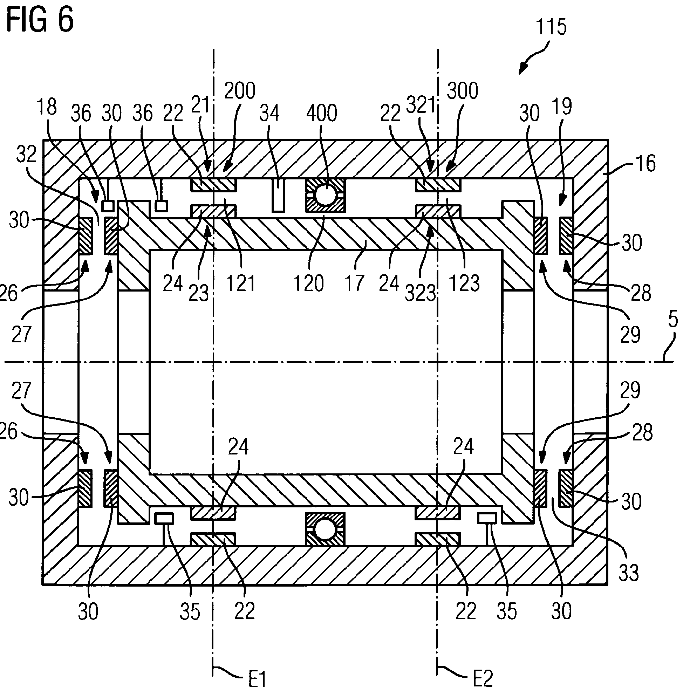

FIG 6 zeigte eine Schnittdarstellung einer zweiten Ausführungsform einer Lager- und Antriebseinheit 115, welche sich von der Lager- und Antriebseinheit 15 dahingehend unterscheidet, dass das Radiallager 200 und die elektromagnetische Antriebseinheit 300 voneinander getrennt und in Richtung der Achse 5 relativ zueinander versetzt angeordnet sind. Außerdem weist die Lager- und Antriebseinheit 115 ein Stützlager 400 auf. Die übrigen Komponenten der Lager- und Antriebseinheit 115 entsprechen in Aufbau und Funktion ggf. mit an die Bauform der Lager- und Antriebseinheit 115 angepassten Abmessungen den Komponenten der Lager- und Antriebseinheit 15, weshalb diese mit gleichen Bezugszeichen versehen sind.

Das Radiallager 200 ist im Falle des in FIG 6 gezeigten Ausführungsbeispiels der Erfindung in einer ersten Ebene E1 angeordnet und weist wie das Mittel zum elektromagnetischen Antrieb umfassende Radiallager 20 eine radial außenliegende ringförmige Radialanordnung 21 und eine radial innenliegende ringförmige Radialanordnung 23 von Elektromagneten und ggf. von Permanentmagneten und/oder von Elementen aus einem ferromagnetischen Material auf.

Die elektromagnetische Antriebseinheit 300 ist in einer in Richtung der Systemachse 5 relativ zu der Ebene E1 versetzten Ebene E2 angeordnet und weist ebenfalls eine radial außenliegende ringförmige Radialanordnung 321 und eine radial innenliegende ringförmige Radialanordnung 323 von Elektromagneten und ggf. von Permanentmagneten und/oder von Elementen aus einem ferromagnetischen Material auf.

Im Unterschied zu dem Mittel zum elektromagnetischen Antrieb umfassenden Radiallager 20 sind alle Elektromagnete und ggf. Permanentmagnete und/oder Elementen aus einem ferromagnetischen Material des Radiallagers 200 für die magnetische Lagerung vorgesehen. Des Weiteren sind alle Elektromagnete und ggf. Permanentmagnete und/oder Elementen aus einem ferromagnetischen Material der elektromagnetischen Antriebseinheit 300 zum elektromagnetischen Antrieb vorgesehen.

Wie bereits erwähnt weist die Lager- und Antriebseinheit 115 zudem ein Stützlager 400 in Form eines konventionellen Wälzlagers auf. Das Stützlager 400 übernimmt die Tragfunktion in der Lager- und Antriebseinheit 115, wenn beispielsweise im Falle eines Stromausfalls das von den Elektromagneten erzeugte Magnetfeld zu schwach wird. Auf diese Weise wird auch eine Beschädigung der Magnetlager der Lager- und Antriebseinheit 115 vermieden. Um diesen Zweck zu erfüllen ist die Weite des Spaltes 120 zwischen dem an dem Trägerelement 16 angeordneten Stützlager 400 und dem Trägerelement 17 kleiner als der Radiallagerspalt 121 und kleiner als der Antriebsspalt 123. Im normalen Betrieb berührt das an dem ringförmigen Trägerelement 16 befestigte Stützlager 400 das ringförmige Trägerelement 17 nicht. Erst im Fehlerfall berührt das Stützlager 400 das ringförmige Trägerelement 17 und übernimmt die Tragfunktion, wodurch auch eine Beschädigung des Radiallagers 200 und der elektromagnetischen Antriebseinheit 300 vermieden wird. Zusätzlich oder alternativ kann die Vorrichtung bzw. das Computertomographiegerät eine unterbrechungsfreie Stromversorgung 124 aufweisen, wie dies in FIG 2 angedeutet ist. Ein Stützlager ist dann, wie für die Lager- und Antriebseinheit 15 gezeigt nicht notwendig, da die Strom- und Spannungsversorgung bei einem Netzausfall durch die unterbrechungsfreie Stromversorgung 124 sichergestellt wird.

## Patentansprüche

1. Vorrichtung (15, 115) zur Lagerung und zum Antrieb eines rotierbaren Teils (4) einer eine Systemachse (5) aufweisenden Gantry (2) eines Computertomographiegerätes (1) relativ zu einem stationären Teil (3) der Gantry (2) des Computertomographiegerätes (1), aufweisend
- Mittel (22, 24, 30) zur magnetischen Lagerung des rotierbaren Teils (4) der Gantry (2) relativ zu dem stationären Teil (3) der Gantry (2) und
- Mittel (22, 24) zum elektromagnetischen Antrieb des rotierbaren Teils (4) der Gantry (2) relativ zu dem stationären Teil (3) der Gantry (2),
wobei die Mittel (22, 24, 30) zur magnetischen Lagerung des rotierbaren Teils (4) der Gantry (2) relativ zu dem stationären Teil (3) der Gantry (2) wenigstens ein Radiallager (20, 200) und wenigstens ein Axiallager (18, 19) bilden und wobei die Mittel (22, 24) zum elektromagnetischen Antrieb des rotierbaren Teils (4) der Gantry (2) relativ zu dem stationären Teil (3) der Gantry (2) in das Radiallager (20) integriert sind.

2. Vorrichtung nach Anspruch 1, bei der die Mittel (22, 24, 30) zur magnetischen Lagerung des rotierbaren Teils (4) der Gantry (2) relativ zu dem stationären Teil (3) der Gantry (2) wenigstens einen Permanentmagneten, wenigstens einen eine Spule aufweisenden Elektromagneten (22, 30) und/oder wenigstens ein Element (24) aus einem ferromagnetischen Material und die Mittel (22, 24) zum elektromagnetischen Antrieb des rotierbaren Teils (4) der Gantry (2) relativ zu dem stationären Teil (3) der Gantry (2) wenigstens einen eine Spule aufweisenden Elektromagneten (22) umfassen.

3. Vorrichtung nach Anspruch 1 oder 2, bei der das Radiallager (20, 200) und/oder die Mittel (22, 24) zum elektromagnetischen Antrieb eine erste, radial außenliegende ringförmige Radialanordnung (21, 321) von Permanentmagneten, Elektromagneten (22) und/oder Elementen aus einem ferromagnetischen Material, welche dem stationären Teil (3) der Gantry (2) zugeordnet ist, und eine zweite radial innenliegende ringförmige Radialanordnung (23, 323) von Permanentmagneten, Elektromagneten und/oder Elementen (24) aus einem ferromagnetischen Material, welche dem rotierbaren Teil (4) der Gantry (2) zugeordnet ist, aufweisen, wobei sich zwischen der ersten und der zweiten ringförmigen Radialanordnung (21, 23) ein ringförmiger Radiallagerspalt (31, 121) befindet.

4. Vorrichtung nach einem der Ansprüche 1 bis 3, bei der das wenigstens eine Axiallager (18, 19) eine erste ringförmige Axialanordnung (26, 28) von Permanentmagneten, Elektromagneten (30) und/oder Elementen aus einem ferromagnetischen Material, welche dem stationären Teil (3) der Gantry (2) zugeordnet ist, und eine zweite ringförmige Axialanordnung (27, 29) von Permanentmagneten, Elektromagneten (30) und/oder Elementen aus einem ferromagnetischen Material, welche dem rotierbaren Teil (4) der Gantry (2) zugeordnet ist, aufweist, welche erste und zweite Axialanordnung (26 bis 29) unter Einschluss eines ringförmigen Axiallagerspalts (32, 33) axial in Richtung der Systemachse (5) relativ zueinander versetzt sind.

5. Vorrichtung nach Anspruch 3 oder 4, welche Messmittel (34, 35, 36) zur Ermittlung der Änderung der Weite des Radiallagerspalts (31, 121) und/oder des Axiallagerspalts (32, 33) aufweist.

6. Vorrichtung nach Anspruch 5, bei der die Messmittel wenigstens einen Hall-Sensor (34, 35, 36) und/oder einen induktiv oder kapazitiv arbeitenden Sensor aufweisen.

7. Vorrichtung nach einem der Ansprüche 2 bis 6, bei der im Falle der Verwendung von Elektromagneten (22, 30) für die magnetische Lagerung die Ermittlung der Änderung der Weite des Radiallagerspalts (31, 121) und/oder des Axiallagerspalts (32, 33) basierend auf der Beeinflussung der Induktivität der Spule eines oder mehrerer Elektromagnete (22, 30) erfolgt.

8. Vorrichtung nach einem der Ansprüche 1 bis 7, bei dem das Radiallager (200) in einer ersten Ebene (E1) und die Mittel (22, 24) zum elektromagnetischen Antrieb des rotierbaren Teils (4) der Gantry (2) relativ zu dem stationären Teil (3) der Gantry (2) in einer zweiten, in Richtung der Systemachse (5) relativ zu der ersten Ebene (E1) versetzten Ebene (E2) angeordnet sind.

9. Vorrichtung nach einem der Ansprüche 1 bis 8, bei der die erste, radial außenliegende ringförmige Radialanordnung (21) Elektromagnete (22) und bei der die zweite radial innenliegende ringförmige Radialanordnung (23) Permanentmagnete und/oder Elementen aus einem ferromagnetischen Material aufweisen, wobei eine Gruppe von Elektromagneten (22) der ersten Radialanordnung (21) derart ansteuerbar ist, dass ein elektromagnetisches Drehfeld zum elektromagnetisches Antrieb des rotierbaren Teils (4) der Gantry (2) erzeugt wird.

10. Vorrichtung nach einem der Ansprüche 1 bis 8, bei der die erste, radial außenliegende ringförmige Radialanordnung (21) Permanentmagnete und/oder Elementen aus einem ferromagnetischen Material und bei der die zweite radial innenliegende ringförmige Radialanordnung (23) Elektromagnete aufweisen, wobei eine Gruppe von Elektromagneten der zweiten Radialanordnung derart ansteuerbar ist, dass ein elektromagnetisches Drehfeld zum elektromagnetisches Antrieb des rotierbaren Teils (4) der Gantry (2) erzeugt wird.

11. Vorrichtung nach einem der Ansprüche 1 bis 8, bei der die erste, radial außenliegende ringförmige Radialanordnung (21) und die zweite radial innenliegende ringförmige Radialanordnung (23) Elektromagnete (22) aufweisen, wobei eine Gruppe von Elektromagneten (22) der ersten und/oder der zweiten Radialanordnung (21, 23) derart ansteuerbar ist, dass ein elektromagnetisches Drehfeld zum elektromagnetisches Antrieb des rotierbaren Teils (4) der Gantry (2) erzeugt wird.

12. Vorrichtung nach einem der Ansprüche 9 bis 11, bei der die zum elektromagnetischen Antrieb und/oder die zur magnetischen Lagerung vorgesehenen Elektromagnete der ersten und/oder der zweiten Radialanordnung (21, 23) jeweils in Segmenten (25) gruppiert sind.

13. Vorrichtung nach einem der Ansprüche 1 bis 12, welche ein Wälzlager als Stützlager (400) aufweist.

14. Vorrichtung nach einem der Ansprüche 1 bis 13, welcher eine unterbrechungsfreie Stromversorgung (124) zugeordnet ist.

15. Computertomographiegerät (1) aufweisend eine Vorrichtung (15, 115) nach einem der Ansprüche 1 bis 14.

## Claims

1. Device (15, 115) for mounting and driving a rotatable part (4) of a gantry (2) of a computed tomography unit (1) relative to a stationary part (3) of the gantry (2) of the computed tomography unit (1), said gantry (2) having a system axis (5), having
- means (22, 24, 30) for magnetically mounting the rotatable part (4) of the gantry (2) relative to the stationary part (3) of the gantry (2) and
- means (22, 24) for electromagnetically driving the rotatable part (4) of the gantry (2) relative to the stationary part (3) of the gantry (2),
wherein the means (22, 24, 30) for the magnetic mounting of the rotatable part (4) of the gantry (2) relative to the stationary part (3) of the gantry (2) form at least one radial bearing (20, 200) and at least one axial bearing (18, 19) and wherein the means (22, 24) for electromagnetically driving the rotatable part (4) of the gantry (2) relative to the stationary part (3) of the gantry (2) are integrated into the radial bearing (20).

2. Device according to claim 1, wherein the means (22, 24, 30) for the magnetic mounting of the rotatable part (4) of the gantry (2) relative to the stationary part (3) of the gantry (2) comprise at least one permanent magnet, at least one electromagnet (22, 30) having a coil and/or at least one element (24) made of a ferromagnetic material, and the means (22, 24) for electromagnetically driving the rotatable part (4) of the gantry (2) relative to the stationary part (3) of the gantry (2) comprise at least one electromagnet (22) having a coil.

3. Device according to claim 1 or 2, wherein the radial bearing (20, 200) and/or the means (22, 24) for the electromagnetic drive have a first, radially outer annular radial arrangement (21, 321) of permanent magnets, electromagnets (22) and/or elements made of a ferromagnetic material, said arrangement (21, 321) being associated with the stationary part (3) of the gantry (2), and a second radially inner annular radial arrangement (23, 323) of permanent magnets, electromagnets, and/or elements (24) made of a ferromagnetic material, said arrangement (23, 323) being associated with the rotatable part (4) of the gantry (2), wherein an annular radial bearing gap (31, 121) is located between the first and the second annular radial arrangement (21, 23).

4. Device according to one of claims 1 to 3, wherein the at least one axial bearing (18, 19) has a first, annular axial arrangement (26, 28) of permanent magnets, electromagnets (30) and/or elements made of a ferromagnetic material, said arrangement (26, 28) being associated with the stationary part (3) of the gantry (2), and a second annular axial arrangement (27, 29) of permanent magnets, electromagnets (30) and/or elements made of a ferromagnetic material, said arrangement (27, 29) being associated with the rotatable part (4) of the gantry (2), which first and second axial arrangements (26 to 29) are offset relative to one another axially in the direction of the system axis (5) and enclose an annular axial bearing gap (32, 33).

5. Device according to claim 3 or 4, which has measuring means (34, 35, 36) for determining the change in the width of the radial bearing gap (31, 121) and/or of the axial bearing gap (32, 33).

6. Device according to claim 5, wherein the measuring means have at least one Hall sensor (34, 35, 36) and/or an inductively or capacitively operating sensor.

7. Device according to one of claims 2 to 6, wherein in the case of the use of electromagnets (22, 30) for the magnetic mounting, the determination of the change in the width of the radial bearing gap (31, 121) and/or of the axial bearing gap (32, 33) takes place on the basis of the effect on the inductance of the coil of one or more electromagnets (22, 30).

8. Device according to one of claims 1 to 7, wherein the radial bearing (200) is arranged in a first plane (El) and the means (22, 24) for electromagnetically driving the rotatable part (4) of the gantry (2) relative to the stationary part (3) of the gantry (2) are arranged in a second plane (E2) offset in the direction of the system axis (5) relative to the first plane (El).

9. Device according to one of claims 1 to 8, wherein the first, radially outer annular radial arrangement (21) comprises electromagnets (22) and wherein the second radially inner annular radial arrangement (23) comprises permanent magnets and/or elements made of a ferromagnetic material, wherein a group of electromagnets (22) of the first radial arrangement (21) can be actuated such that an electromagnetic rotary field is generated for electromagnetically driving the rotatable part (4) of the gantry (2).

10. Device according to one of claims 1 to 8, wherein the first, radially outer annular radial arrangement (21) comprises permanent magnets and/or elements made of a ferromagnetic material and wherein the second radially inner annular radial arrangement (23) comprises electromagnets, wherein a group of electromagnets of the second radial arrangement can be actuated such that an electromagnetic rotary field is generated for electromagnetically driving the rotatable part (4) of the gantry (2).

11. Device according to one of claims 1 to 8, wherein the first, radially outer annular radial arrangement (21) and the second radially inner annular radial arrangement (23) comprise electromagnets (22), wherein a group of electromagnets (22) of the first and/or of the second radial arrangement (21, 23) can be actuated such that an electromagnetic rotary field is generated for electromagnetically driving the rotatable part (4) of the gantry (2).

12. Device according to one of claims 9 to 11, wherein the electromagnets of the first and/or of the second radial arrangement (21, 23) which are provided for the electromagnetic drive and/or for the magnetic mounting are each grouped into segments (25).

13. Device according to one of claims 1 to 12, which has a rolling bearing as a support bearing (400).

14. Device according to one of claims 1 to 13, with which an uninterruptible power supply (124) is associated.

15. Computed tomography device (1) having a device (15, 115) according to one of claims 1 to 14.

## Revendications

1. Dispositif (15, 115) de montage et d'entraînement d'une partie (4) tournante d'un portique (2), ayant un axe (5) de système, d'un tomodensitomètre (1) par rapport à une partie (3) fixe du portique (2) du tomodensitomètre (1) comportant
- des moyens (22, 24, 30) de montage magnétique de la partie (4) tournante du portique (2) par rapport à la partie (3) fixe du portique (2) et
- des moyens (22, 24) d'entraînement électromagnétique de la partie (4) tournante du portique (2) par rapport à la partie (3) fixe du portique (2),
les moyens (22, 24, 30) de montage magnétique de la partie (4) tournante du portique (2) par rapport à la partie (3) fixe du portique (2) formant au moins un palier (20, 200) radial et au moins un palier (18, 19) axial et dans lequel les moyens (22, 24) d'entraînement électromagnétique de la partie (4) tournante du portique par rapport à la partie (3) fixe du portique (2) sont intégrés dans le palier (20) radial.

2. Dispositif suivant la revendication 1, dans lequel les moyens (22, 24, 30) de montage magnétique de la partie (4) tournante du portique (2) par rapport à la partie (3) fixe du portique (2) comprennent au moins un aimant permanent, au moins un électroaimant (22, 30) ayant une bobine et au moins un élément en matériau ferromagnétique et les moyens (22, 24) d'entraînement électromagnétique de la partie (4) tournante du portique (2) par rapport à la partie (3) fixe du portique (2) comprennent au moins un électroaimant (22) ayant une bobine.

3. Dispositif suivant la revendication 1 ou 2, dans lequel le palier (20, 200) radial et/ou les moyens (22, 24) d'entraînement électromagnétique ont un premier agencement (21, 321) radial annulaire se trouvant à l'extérieur radialement d'aimants permanents, d'électroaimants (22) et/ou d'éléments en matériau ferromagnétique, premier agencement qui est associé à la partie (3) fixe du portique (2), et un deuxième agencement (23, 323) radial annulaire se trouvant à l'intérieur radialement d'aimants permanents, d'électroaimants et/ou d'éléments (24) en un matériau ferromagnétique, deuxième agencement qui est associé à la partie (4) tournante du portique (2), un intervalle (31, 321) annulaire de palier radial se trouvant entre le premier et le deuxième agencement (21, 23) radial annulaire.

4. Dispositif suivant la revendication 1 à 3, dans lequel le au moins un palier (18, 19) axial a un premier agencement (26, 28) axial annulaire d'aimants permanents, d'électroaimants (30) et/ou d'éléments en un matériau ferromagnétique, premier agencement (26, 28) qui est associé à la partie (3) fixe du portique (2), et un deuxième agencement (27, 29) axial annulaire d'aimants permanents, d'électroaimants (30) et/ou d'éléments en un matériau ferromagnétique, deuxième agencement qui est associé à la partie (4) tournante du portique (2), lesquels premier et deuxième agencements (26 à 29) axiaux sont, par inclusion d'un intervalle (32, 33) annulaire de palier axial, décalés l'un par rapport à l'autre axialement dans la direction de l'axe (5) du système.

5. Dispositif suivant la revendication 3 ou 4, qui a des moyens (34, 35, 36) de mesure pour déterminer la variation de la largeur de l'intervalle (31, 121) de palier radial et/ou de l'intervalle (32, 33) de palier axial.

6. Dispositif suivant la revendication 5, dans lequel les moyens de mesure ont au moins un capteur (34, 35, 36) de Hall et/ou un capteur à fonctionnement inductif ou capacitif.

7. Dispositif suivant l'une des revendications 2 à 6, dans lequel, dans le cas où l'on utilise des électroaimants (22, 30) pour le montage magnétique, on effectue la détermination de la variation de la largeur de l'intervalle (31, 121) de palier radial et/ou de l'intervalle (32, 33) de palier axial sur la base de l'influence de l'inductance de la bobine d'un ou de plusieurs électroaimants (22, 30).

8. Dispositif suivant l'une des revendications 1 à 7, dans lequel le palier (200) radial est dans un premier plan (E1) et les moyens (22, 24) d'entraînement électromagnétique de la partie (4) tournante du portique (2) par rapport à la partie (3) fixe du portique (2) sont dans un deuxième plan (E2) décalé par rapport au premier plan (E1) dans la direction de l'axe (5) du système.

9. Dispositif suivant l'une des revendications 1 à 8, dans lequel le premier agencement (21) radial annulaire se trouvant à l'extérieur radialement a des électroaimants (22) et le deuxième agencement (23) radial annulaire se trouvant à l'intérieur radialement a des aimants permanents et/ou des éléments en un matériau ferromagnétique, un groupe d'électroaimants (22) du premier agencement (21) radial pouvant être excité de manière à produire un champ électromagnétique tournant pour l'entraînement électromagnétique de la partie (4) tournante du portique (2).

10. Dispositif suivant l'une des revendications 1 à 8, dans lequel le premier agencement (21) radial annulaire se trouvant à l'extérieur radialement a des aimants permanents et/ou des éléments en un matériau ferromagnétique et le deuxième agencement (23) radial annulaire se trouvant à l'intérieur radialement a des électroaimants, un groupe d'électroaimants du deuxième agencement radial pouvant être excité de manière à produire un champ tournant électromagnétique pour l'entraînement électromagnétique de la partie (4) tournante du portique (2).

11. Dispositif suivant l'une des revendications 1 à 8, dans lequel le premier agencement (21) radial annulaire se trouvant à l'extérieur radialement et le deuxième agencement (23) radial annulaire se trouvant à l'intérieur radialement ont des électroaimants (22), un groupe d'électroaimants (22) du premier et/ou du deuxième agencements (21, 23) radial pouvant être excité de manière à produire un champ tournant électromagnétique pour l'entraînement électromagnétique de la partie (4) tournante du portique (2).

12. Dispositif suivant l'une des revendications 9 à 11, dans lequel les électroaimants prévus pour l'entraînement électromagnétique et/ou pour le montage magnétique du premier et/ou du deuxième agencements (21, 23) radial sont regroupés respectivement en des segments (25).

13. Dispositif suivant l'une des revendications 1 à 12, qui a un palier à roulement comme palier (400) d'appui.

14. Dispositif suivant l'une des revendications 1 à 13, auquel est associé une alimentation (124) de courant ininterrompue.

15. Tomodensitomètre (1) ayant un dispositif (15, 115) suivant l'une des revendications 1 à 14.
